# EUROPEAN PATENT APPLICATION

(11) **EP 4 837 954 A1**
(43) Date of publication of application: **11.11.2026**
(21) Application number: 25741627.1
(22) Date of filing: 17.01.2025
(51) Int. Cl.: C12M 1/42, C12M 1/36, C12M 1/34, C12M 1/00, C12Q 1/6869

(54) **SENSOR DEVICE AND METHOD FOR OPERATING SENSOR DEVICE**

(30) Priority: 17.01.2024 CN 202410068766
(71) Applicant: Qitan Technology Ltd., Beijing, Beijing 100192 (CN)
(72) Inventor: E, Qiang, Beijing 100192 (CN); WANG, Jin, Beijing 100192 (CN); LIU, Zhao, Beijing 100192 (CN)
(74) Representative: Boult Wade Tennant LLP
(86) International application number: PCT/CN2025/073029
(87) International publication number: WO 2025/153065

(57) **Abstract**

The present application discloses a sensor device and a method for operating the sensor device. The sensor device comprises: a chamber; a first electrode connected to a common voltage end, wherein the potential difference between the first electrode and a corresponding second electrode is used for driving an analyte to be detected to penetrate through a corresponding nanopore and generate a sensing electrical signal; a bias voltage generation module used for generating a bias voltage less than or equal to a common voltage provided by the common voltage end; a first multi-path selection module, wherein the first multi-path selection module is connected to a plurality of second electrodes and can select one of the second electrodes to output a sensing electrical signal while the rest second electrodes are connected to the bias voltage generation module or the common voltage end; and a signal processing module, wherein the signal processing module is connected to the first multi-path selection module and can obtain the state of the nanopore on the basis of the sensing electrical signal.

## Description

### CROSS-REFERENCE TO RELATED APPLICATIONS

The present application claims priority to Chinese Patent Application No. 202410068766.7 filed on January 17, 2024 and entitled "SENSOR DEVICE AND METHOD FOR OPERATING SENSOR DEVICE", which is incorporated herein by reference in its entirety.

### TECHNICAL FIELD

The present application relates to the technical field of biological detection, and in particular, to a sensor device and a method for operating the sensor device.

### BACKGROUND

In nanopore sequencing, a biopolymer is driven to pass through a nanopore sequencing channel on a membrane by applying a certain potential, and the biopolymer passing through the nanopore sequencing channel is identified by utilizing the current change caused by different molecules when passing through the nanopore sequencing channel.

Taking single-stranded DNA as an example, a membrane can divide a chamber into two chambers. In the case that there is an electrode in each of the two chambers and the two electrodes have different potentials, the single-stranded DNA can be driven to pass through a nanopore. When the single-stranded DNA passes through the nanopore, the blockage of the nanopore on the membrane by bases will increase the resistance effect of the nanopore on the current between the electrodes, i.e., change the equivalent resistance value of the nanopore. Due to the difference in properties of different bases, the degree of blockage of the nanopore on the membrane by different bases is different; that is, the degree of change in the equivalent resistance value of the nanopore is also different. The gene sequence information of the single-stranded DNA can be obtained by reading the current change between the electrodes.

In the nanopore sequencing process, in order to improve the efficiency of nanopore gene sequencing and reduce the influence of nanopore damage of the membrane on the sequencing, a design scheme of a redundant nanopore array is usually adopted; that is, one of a plurality of nanopores on the membrane can be selected to be connected to a detection channel for gene sequencing. However, there may be charge accumulation in the remaining nanopores that are not connected to the detection channel, which leads to generation of a pulse current, thereby affecting the accuracy of the nanopore sequencing and even causing damage to the membrane and the nanopores.

### SUMMARY

Embodiments of the present application provide a sensor device and a method for operating the sensor device, which can solve the technical problem that unused nanopores cause charge accumulation and thereby affect nanopore sequencing.

In a first aspect, the embodiments of the present application provide a sensor device. The sensor device includes:
a chamber, where in a case that the chamber is provided with a membrane including a plurality of nanopores, the chamber is divided into a first chamber and a second chamber by the membrane, the second chamber includes a plurality of second sub-chambers separated from each other, and each of the second sub-chambers is in communication with the first chamber through a corresponding nanopore;
a first electrode, disposed in the first chamber, where the first electrode is connected to a common voltage terminal;
a plurality of second electrodes, disposed in the plurality of second sub-chambers, respectively, where a potential difference between the first electrode and each of the second electrodes is used to drive an analyte to be detected to pass through a corresponding nanopore and generate a sensing electrical signal;
a bias voltage generation module, configured to generate a bias voltage, where the bias voltage is less than or equal to a common voltage provided by the common voltage terminal;
a first multiplex selection module, connected to the plurality of second electrodes, where the first multiplex selection module is capable of selecting one second electrode from the plurality of second electrodes to be in communication with an output terminal of the first multiplex selection module to output a sensing electrical signal, and connecting second electrodes that are not in communication with the output terminal of the first multiplex selection module to the bias voltage generation module or the common voltage terminal; and
a signal processing module, where an input terminal of the signal processing module is connected to the first multiplex selection module, and the signal processing module is capable of acquiring a state of a nanopore according to a sensing electrical signal output by the first multiplex selection module.

In some embodiments, the first multiplex selection module includes:
a first switch module, where a plurality of first terminals of the first switch module are connected to the plurality of second electrodes, respectively, and a second terminal of the first switch module is connected to the output terminal of the first multiplex selection module; the first switch module is configured to output a sensing electrical signal of one of the plurality of second electrodes; and
a second switch module, where a plurality of first terminals of the second switch module are connected to the plurality of second electrodes, respectively, and a second terminal of the second switch module is connected to the bias voltage generation module.

In some embodiments, the first switch module includes:
a plurality of sensing switches, where each of the sensing switches is connected between a corresponding second electrode and the output terminal of the first multiplex selection module;
at any time, at most one of the plurality of sensing switches is in an ON state;
the second switch module includes:
   a plurality of first bias switches, where each of the first bias switches is connected between a corresponding second electrode and the bias voltage generation module.

In some embodiments, a third terminal of the second switch module is connected to the common voltage terminal; the second switch module further includes:
a plurality of second bias switches, where each of the second bias switches is connected between a corresponding second electrode and the common voltage terminal, where the bias voltage generated by the bias voltage generation module is less than the common voltage provided by the common voltage terminal.

In some embodiments, the sensor device further includes:
a signal detection circuit, where the signal detection circuit is connected between the first multiplex selection module and the signal processing module, and is configured to perform signal amplification on a sensing electrical signal from the first multiplex selection module.

In some embodiments, the sensor device includes a plurality of first multiplex selection modules; the signal detection circuit includes:
a plurality of current amplification units, where an input terminal of each of the current amplification units is connected to a corresponding first multiplex selection module; the current amplification unit is configured to perform signal amplification on a sensing electrical signal output by the corresponding first multiplex selection module;
the sensor device further includes:
   a second multiplex selection module, where the second multiplex selection module is connected to output terminals of the plurality of current amplification units, and is configured to output an amplified sensing electrical signal from one of the plurality of current amplification units; and an analog-to-digital conversion module, where an input terminal of the analog-to-digital conversion module is connected to an output terminal of the second multiplex selection module, and an output terminal of the analog-to-digital conversion module is connected to the input terminal of the signal processing module.

In some embodiments, the sensor device further includes:
a controller module, where an input terminal of the controller module is connected to an output terminal of the signal processing module, and a gate signal terminal of the controller module is connected to the first multiplex selection module; the controller module is configured to generate a gate signal corresponding to one of the plurality of second electrodes and send the gate signal to the first multiplex selection module.

In some embodiments, the controller module is connected to the bias voltage generation module.

In a case that there is at least one nanopore in a damaged state and the second switch module does not include a second bias switch, the controller module is configured to drive the bias voltage generation module to generate a bias voltage equal to the common voltage.

In some embodiments, in a case that there is at least one nanopore in a damaged state and the second switch module includes a second bias switch, the controller module is configured to drive the first multiplex selection module to switch a second electrode corresponding to the nanopore in the damaged state to be connected to the common voltage terminal.

In a second aspect, the embodiments of the present application provide a method for operating a sensor device, applied to the sensor device according to the first aspect. The method includes: acquiring states of the plurality of nanopores, and determining a target nanopore according to the states of the plurality of nanopores; and
electrically connecting a second electrode corresponding to the target nanopore to the signal processing module, and connecting second electrodes corresponding to remaining nanopores to the bias voltage generation module or the common voltage terminal, so as to analyze and process an analyte to be detected through a sensing electrical signal generated when the analyte to be detected passes through the target nanopore.

In some embodiments, acquiring the states of the plurality of nanopores and determining the target nanopore according to the states of the plurality of nanopores, includes:
enabling the first multiplex selection module to sequentially electrically connect a second electrode corresponding to each nanopore to the signal processing module;
determining a state of each nanopore according to a sensing electrical signal corresponding to each nanopore; and
selecting a nanopore in a normal state as the target nanopore.

In some embodiments, the method for operating a sensor device further includes:
monitoring, according to a sensing electrical signal generated by the target nanopore, a state of the target nanopore in a case that the second electrode corresponding to the target nanopore is electrically connected to the signal processing module; and
when the target nanopore is damaged, switching the second electrode corresponding to the target nanopore to be connected to the bias voltage generation module or the common voltage terminal.

Compared with the related art, the sensor device and the method for operating the sensor device provided in the embodiments of the present application can generate a bias voltage less than or equal to a common voltage by providing the bias voltage generation module. When the first multiplex selection module selects one second electrode from the plurality of second electrodes to be in communication with the output terminal of the first multiplex selection module to output a sensing electrical signal, the first multiplex selection module can connect remaining second electrodes to the bias voltage generation module or the common voltage terminal. In this case, the nanopore corresponding to the selected second electrode can generate a sensing electrical signal when the analyte to be detected passes through the nanopore, and the signal processing module can analyze and process the analyte to be detected according to the sensing electrical signal. The second electrodes corresponding to other redundant nanopores can receive a bias voltage or a common voltage instead of being in a floating state, thereby alleviating the problem that a pulse current may be caused by the charge accumulation on the nanopores of the membrane due to the second electrodes of the redundant nanopores in the floating state, and improving the stability and safety of the sensor device. In addition, when the bias voltage is set to be less than the common voltage, the consumed electrode or electrolyte of the redundant nanopore can be repaired through a reduction reaction, thereby prolonging the service life of the electrode or electrolyte.

### BRIEF DESCRIPTION OF THE DRAWINGS

To more clearly illustrate the technical solutions in embodiments of the present application, the drawings required for illustrating the embodiments of the present application are briefly described below. Apparently, the drawings in the following description illustrate merely some embodiments of the present application, and those of ordinary skill in the art may still derive other drawings from these drawings without creative efforts.
FIG. 1 is a schematic structural view of a module of a sensor device according to one embodiment of the present application;
FIG. 2 is a schematic structural view of a part of a sensor device according to another embodiment of the present application;
FIG. 3 is a schematic structural view of a circuit of a first multiplex selection module according to one embodiment of the present application;
FIG. 4 is a schematic structural view of a module of a sensor device according to another embodiment of the present application;
FIG. 5 is a schematic structural view of a circuit of a first multiplex selection module according to another embodiment of the present application;
FIG. 6 is a schematic structural view of a module of a sensor device according to yet another embodiment of the present application;
FIG. 7 is a schematic structural view of a module of a sensor device according to still another embodiment of the present application;
FIG. 8 is a schematic flowchart of a method for operating a sensor device according to one embodiment of the present application;
FIG. 9 is a schematic flowchart of a method for operating a sensor device according to another embodiment of the present application; and
FIG. 10 is a schematic view of a voltage change in a bias voltage in a nanopore switching process according to one embodiment of the present application.

In the drawings:
e1, first electrode; e2, second electrode; np, nanopore; 10, first multiplex selection module; 11, first switch module; 12, second switch module; 20, bias voltage generation module; 30, signal processing module; 40, signal detection circuit; 41, current amplification unit; 42, second multiplex selection module; 43, analog-to-digital conversion module; 50, controller module; VCOM, common voltage terminal; S1, sensing switch; S21, first bias switch; S22, second bias switch.

### DETAILED DESCRIPTION

Features and exemplary embodiments of various aspects of the present application will be described in detail below. To make the objectives, technical solutions, and advantages of the present application more apparent, the present application is further described in detail below with reference to the drawings and specific embodiments. It should be understood that the specific embodiments described herein are intended only to illustrate the present application, and not to limit the present application. For those skilled in the art, the present application can be implemented without some of these specific details. The following description of the embodiments is intended only to provide a better understanding of the present application by illustrating examples of the present application.

It should be noted that relational terms herein, such as first and second, are merely used to distinguish one entity or operation from another entity or operation without necessarily requiring or implying any such actual relationship or order between these entities or operations. Moreover, the terms "comprise", "include", or any other variants thereof are intended to cover a non-exclusive inclusion, such that a process, method, article, or device that includes a list of elements includes not only those elements but also other elements not explicitly listed or inherent to such process, method, article, or device. In the absence of further limitations, an element limited by the phrases "comprising ..." and "including ..." does not exclude the presence of additional identical elements in a process, method, article, or device that comprises/includes the element.

It should be noted that in the absence of any conflict, the embodiments in the present application and the features in the embodiments may be combined with each other. The embodiments will be described in detail below with reference to the drawings.

In nanopore sequencing, a biopolymer is driven to pass through a nanopore sequencing channel on a membrane by applying a certain potential, and the biopolymer passing through the nanopore sequencing channel is identified by utilizing the current change caused by different molecules when passing through the nanopore sequencing channel.

Taking single-stranded DNA as an example, a membrane can divide a chamber into two chambers. In the case that there is an electrode in each of the two chambers and the two electrodes have different potentials, the single-stranded DNA can be driven to pass through a nanopore. When the single-stranded DNA passes through the nanopore, the blockage of the nanopore on the membrane by bases will increase the resistance effect of the nanopore on the current between the electrodes, i.e., change the equivalent resistance value of the nanopore. Due to the difference in properties of different bases, the degree of blockage of the nanopore on the membrane by different bases is different; that is, the degree of change in the equivalent resistance value of the nanopore is also different. The gene sequence information of the single-stranded DNA can be obtained by reading the current change between the electrodes.

In the nanopore sequencing process, in order to improve the efficiency of nanopore gene sequencing and reduce the influence of nanopore damage of the membrane on the sequencing, a design scheme of a redundant nanopore array is usually adopted; that is, one of a plurality of nanopores on the membrane can be selected to be connected to a detection channel for gene sequencing. However, there may be charge accumulation in the remaining nanopores that are not connected to the detection channel, which leads to generation of a pulse current, thereby affecting the accuracy of the nanopore sequencing and even causing damage to the membrane and the nanopores.

To solve the technical problems described above, the embodiments of the present application provide a sensor device and a method for operating the sensor device. The sensor device provided in the embodiments of the present application is first described below.

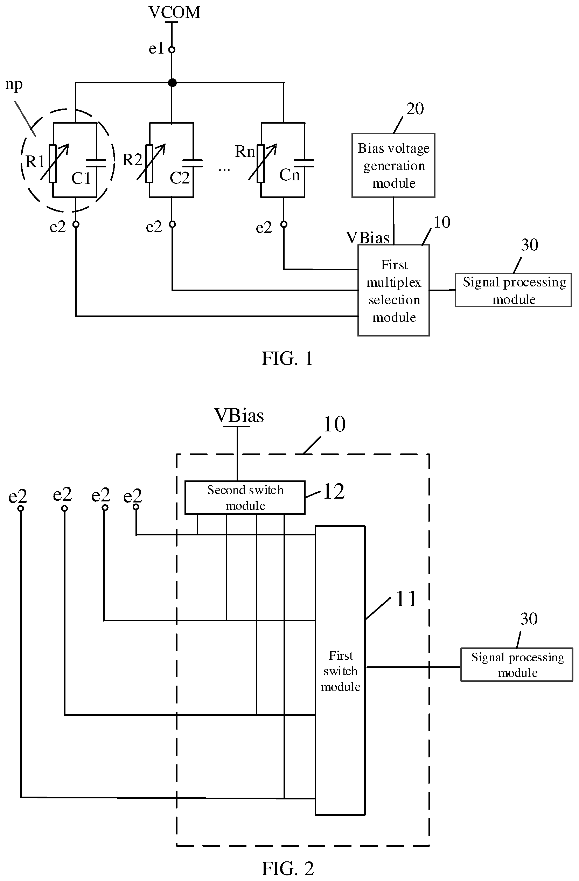

FIG. 1 shows a schematic structural view of a sensor device according to one embodiment of the present application. The sensor device includes a chamber, a first electrode e1, a plurality of second electrodes e2, a bias voltage generation module 20, a first multiplex selection module 10, and a signal processing module 30.

The chamber (not shown) may be provided with a membrane (not shown) including a plurality of nanopores np. When the chamber is provided with a membrane including a plurality of nanopores np, the membrane including the plurality of nanopores np can divide the chamber into a first chamber and a second chamber. The second chamber includes a plurality of second sub-chambers separated from each other, and each of the second sub-chambers may be in communication with the first chamber through a corresponding nanopore np.

The first electrode e1 may be disposed in the first chamber, and the first electrode e1 is connected to a common voltage terminal VCOM.

The plurality of second electrodes e2 may be disposed in the plurality of second sub-chambers, respectively. Each nanopore np on the membrane can serve as a channel between the first chamber and the second sub-chamber, such that an analyte to be detected can pass through the nanopore np from the first chamber to the second sub-chamber.

Each nanopore np can correspond to the first electrode e1 and one second electrode e2. As shown in FIG. 1, each nanopore np may be equivalent to a parallel connection of a resistor and a capacitor. The resistance is the equivalent resistance of the nanopore np, and the capacitance is the equivalent parallel parasitic capacitance of a membrane position where the nanopore np is located. A first end of each nanopore np may be connected to the first electrode e1, and a second end of each nanopore np is connected to a corresponding second electrode e2.

The first electrode e1 is connected to the common voltage terminal VCOM, and the common voltage terminal VCOM can provide a common voltage, such that a potential of the first electrode e1 is the common voltage.

Taking a single nanopore np as an example, there is a potential difference between the first electrode e1 and the second electrode e2 corresponding to the nanopore np, and the potential difference can drive the analyte to be detected in the first chamber to pass through the nanopore np to the second sub-chamber and generate a sensing electrical signal in the process of passing through the nanopore np.

The nanopore np on the membrane can be regarded as an equivalent resistance, and in the process of the analyte to be detected passing through the nanopore np, bases of the analyte to be detected block the nanopore np, thereby changing the equivalent resistance value of the nanopore np. That is, when the analyte to be detected passes through the nanopore np, the nanopore np may be equivalent to a variable resistance. Since the influence of different bases on the equivalent resistance value of the nanopore np is different, the corresponding four bases, A, T, C, and G, can be determined by detecting the change of the sensing electrical signal when the analyte to be detected passes through the nanopore np. Taking single-stranded DNA as an example, the gene sequencing of the single-stranded DNA can be achieved when the single-stranded DNA passes through the nanopore np.

The bias voltage generation module 20 may generate a bias voltage through a bias voltage terminal VBias. A voltage value of the bias voltage may be set to be consistent with the common voltage provided by the common voltage terminal VCOM, or the bias voltage may be less than the common voltage. As an optional implementation, the bias voltage generation module 20 described above may be a digital-to-analog conversion module.

The first multiplex selection module 10 may be connected to the plurality of second electrodes e2. The first multiplex selection module 10 can select one second electrode e2 from the plurality of second electrodes e2, and communicate the second electrode e2 with an output terminal of the first multiplex selection module 10 to output a sensing electrical signal.

The first multiplex selection module 10 may further connect the second electrodes e2 that are not in communication with the output terminal of the first multiplex selection module 10 to the bias voltage generation module 20 or the common voltage terminal VCOM. That is, among the plurality of second electrodes e2 connected to the first multiplex selection module 10, at most one second electrode e2 is in communication with the output terminal of the first multiplex selection module 10, and the remaining second electrodes e2 are connected to the bias voltage generation module 20 or the common voltage terminal VCOM.

An input terminal of the signal processing module 30 is connected to the first multiplex selection module 10. When the first multiplex selection module 10 outputs a sensing electrical signal through the output terminal, the signal processing module 30 can receive the sensing electrical signal output by the first multiplex selection module 10 and acquire a state of the nanopore np according to the sensing electrical signal.

The state of the nanopore np may be a normal state or a damaged state. The signal processing module 30 may acquire the state of the nanopore np according to the sensing electrical signal. When the state of the nanopore np is a damaged state, the nanopore np is determined to be damaged; when the state of the nanopore np is a normal state, the signal processing module 30 can analyze and process the analyte to be detected according to the sensing electrical signal, and obtain a corresponding analysis result. For example, the signal processing module 30 may determine a gene sequence of the analyte to be detected according to the sensing electrical signal.

It should be noted that among the plurality of second electrodes e2, the nanopore np corresponding to the second electrode e2 in communication with the output terminal of the first multiplex selection module 10 is the nanopore np where the analyte to be detected is analyzed and processed, while the remaining nanopores np serve as redundant nanopores np and are not subjected to analysis and processing at this time.

If the second electrodes e2 of the remaining redundant nanopores np are in a floating state when no sequencing is performed, the equivalent resistance values corresponding to the nanopores np are large, and usually the resistance values of the nanopores np in the floating state can reach the GΩ level. Since the plurality of nanopores np are all in the floating state, the surface of the membrane will be affected by the sensing electrical signal to cause charge accumulation, and the accumulated charge cannot be bled due to lack of an effective charge bleeding channel.

When the nanopore np where the analyte to be detected is analyzed and processed is switched, once the second electrode e2 of the switched nanopore np is in communication with the output terminal of the first multiplex selection module 10, the accumulated charge in the switched nanopore may generate a large instantaneous pulse current during bleeding, thereby affecting the analysis and processing process, and even the pulse current may cause damage to the nanopore np on the membrane. Therefore, the redundant nanopores np should avoid being in a floating state as much as possible when not subjected to analysis and processing.

In the above embodiments, except for the second electrode e2 connected to the output terminal of the first multiplex selection module 10, other second electrodes e2 may be connected to the bias voltage generation module 20 or the common voltage terminal VCOM. That is, the second electrode e2 of each redundant nanopore np is not in a floating state, but receives a bias voltage or a common voltage.

Since the second electrode e2 of each redundant nanopore np can receive a common voltage or a bias voltage, a charge bleeding channel can exist in each redundant nanopore np, and there will not be excessive charge accumulation when no analysis or processing is performed. When the nanopore np is switched, no large pulse current is generated since the charge accumulation in the redundant nanopore np is small.

It should be noted that in order to enable the analyte to be detected to move from the first chamber to the second chamber, the potential of the first electrode e1 should be set to be at least not less than the potential of the second electrode e2; that is, the common voltage provided by the common voltage terminal VCOM is greater than or equal to the bias voltage generated by the bias voltage generation module 20.

When the common voltage is consistent with the bias voltage, the potentials between the first electrode e1 and the second electrode e2 of the redundant nanopore np are equal. In this case, the first electrode e1 and the second electrode e2 do not drive the analyte to be detected to pass through the redundant nanopore np.

When the common voltage is greater than the bias voltage, the electrode or the electrolyte can also be repaired.

Taking an oxidizable electrode material as an example, in the nanopore np sequencing process, the second electrode will be connected to a potential less than the common voltage where the first electrode is located, and the electrode in the second chamber will be continuously oxidized. For example, when the electrode material is silver, the oxidation process of the electrode is as follows:

Ag → Ag⁺ + e.

That is, the second electrode e2 will be oxidized into silver ions and electrons, resulting in electrode loss in the nanopore np sequencing process.

Similarly, when the electrode is made of an inert material, there will be no electrode loss in the nanopore np sequencing process. However, in order to generate a sensing electrical signal, an electrolyte needs to be injected into the second chamber; in this case, ions in the electrolyte will be consumed continuously in the nanopore np sequencing process. For example, when the electrode material is platinum and the electrolyte is a potassium ferrocyanide (K4[Fe(CN)6]) solution, although there will be no electrode loss in the sequencing process, iron ions in the electrolyte will be continuously oxidized as follows:

Fe²⁺ → Fe³⁺ + e.

As can be seen from the above examples, whether an oxidizable electrode or an electrolyte is used to provide a sensing electrical signal, the electrode or the electrolyte is consumed in the nanopore np sequencing process.

When Ag at the electrode or Fe²⁺ ions in the electrolyte are nearly completely consumed due to continuous oxidation, the sensing electrical signal is significantly reduced, and the nanopore np sequencing cannot be continued. Therefore, in the nanopore np sequencing process, if a certain nanopore np has been subjected to sequencing for a period of time and the electrode or electrolyte consumption is serious, such that the sensing electrical signal is reduced to be outside the measurable range, the nanopore np subjected to sequencing can be switched, and the electrode or electrolyte consumed in the sequencing process can be repaired.

In the above embodiments, when the bias voltage is set to be less than the common voltage, the consumed electrode or electrolyte can be repaired. For example, when an oxidizable electrode is used as the electrode, the electrode will be continuously oxidized in the sequencing process. For the redundant nanopore np, the second electrode will be connected to a bias voltage less than the common voltage and undergo a reduction reaction. Taking silver as the electrode material as an example, the electrode reduction process is as follows:

Ag⁺ + e → Ag.

Similarly, when the electrode material is an inert material, such as platinum, and the electrolyte is a potassium ferrocyanide solution, the reduction process of iron ions in the electrolyte is as follows:

Fe³⁺ + e → Fe²⁺.

Through the reduction processes described above, when the electrode is consumed or ions in the electrolyte are consumed due to previous nanopore np sequencing performed on the redundant nanopore np, the reduction process of the electrode or the reduction process of the ions in the electrolyte can be achieved by means of the potential difference between the common voltage and the bias voltage, such that the metal electrode or electrolyte consumed during sequencing can be repaired, thereby prolonging the service life of the electrode or electrolyte.

In this embodiment, by providing the bias voltage generation module 20, a bias voltage less than or equal to a common voltage can be generated. When the first multiplex selection module 10 selects one second electrode e2 from the plurality of second electrodes e2 to be in communication with the output terminal of the first multiplex selection module 10 to output a sensing electrical signal, the first multiplex selection module can connect remaining second electrodes e2 to the bias voltage generation module 20 or the common voltage terminal VCOM. The nanopore np corresponding to the selected second electrode e2 can generate a sensing electrical signal when the analyte to be detected passes through the nanopore, and the signal processing module 30 can analyze and process the analyte to be detected according to the sensing electrical signal. The other redundant nanopores np can be connected to the bias voltage generation module 20 or the common voltage terminal VCOM by means of the first multiplex selection module 10. The second electrodes e2 corresponding to the redundant nanopores np can receive a bias voltage or a common voltage instead of being in a floating state, thereby alleviating the problem that a pulse current may be caused by the charge accumulation on the nanopores np of the membrane due to the second electrodes e2 of the redundant nanopores np in the floating state, and improving the stability and safety of the sensor device. In addition, when the bias voltage is set to be less than the common voltage, the consumed electrode or electrolyte of the redundant nanopore np can be repaired through a reduction reaction, thereby prolonging the service life of the electrode or electrolyte.

It should be noted that when the bias voltage is set to be less than the common voltage and the redundant nanopore np is previously connected to the output terminal of the first multiplex selection module 10 for sequencing, the consumed electrode or electrolyte can be repaired. If the redundant nanopore np is not previously connected to the output terminal of the first multiplex selection module 10 and there is no consumption, connecting the second electrode e2 thereof to the bias voltage less than the common voltage will not cause repair or have adverse effects.

Referring to FIG. 2, in some embodiments, the first multiplex selection module 10 described above may include a first switch module 11 and a second switch module 12.

A plurality of first terminals of the first switch module 11 can be connected to the plurality of second electrodes e2, respectively, and a second terminal of the first switch module 11 is connected to the output terminal of the first multiplex selection module 10. The first switch module 11 can connect one of the plurality of second electrodes e2 to the output terminal of the first multiplex selection module 10 to output a sensing electrical signal of the one second electrode e2 of the plurality of second electrodes e2.

A plurality of first terminals of the second switch module 12 can be connected to the plurality of second electrodes e2, respectively, and a second terminal of the second switch module 12 can be connected to the bias voltage generation module 20. The second switch module 12 can connect each of the remaining second electrodes e2 except for the second electrode e2 connected to the output terminal of the first multiplex selection module 10 among the plurality of second electrodes e2 to the bias voltage generation module 20 to provide a bias voltage to each of the remaining second electrodes e2, so as to avoid each of the remaining second electrodes e2 from being in a floating state.

By means of the first switch module 11 and the second switch module 12, one of the plurality of nanopores np can be selected for operation. One of the plurality of nanopores np can output a sensing electrical signal through a corresponding second electrode e2, while the second electrodes e2 of the other redundant nanopores np not under operation can receive a bias voltage, thereby ensuring that a charge bleeding channel exists in the membrane where each of the redundant nanopores np is located, and avoiding excessive charge accumulation on the surface of the membrane.

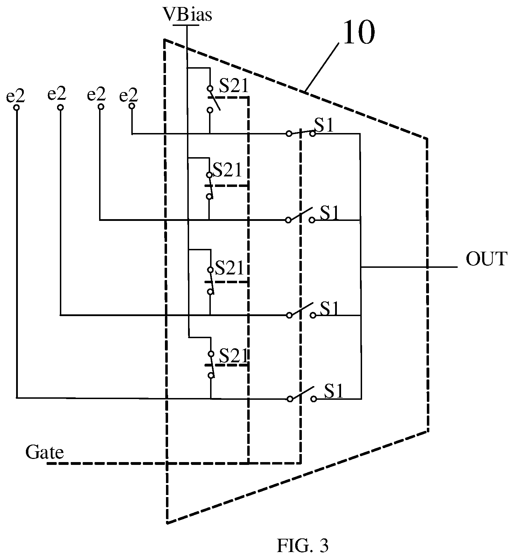

Referring to FIG. 3, in some embodiments, the first switch module 11 described above may include a plurality of sensing switches S1, and the second switch module 12 may include a plurality of first bias switches S21.

Among the plurality of sensing switches S1, each sensing switch S1 can be connected between a corresponding second electrode e2 and the output terminal of the first multiplex selection module 10. When the sensing switch S1 is turned on, it is equivalent to communicating the corresponding second electrode e2 with the output terminal of the first multiplex selection module 10.

At any time, at most one of the plurality of sensing switches S1 is in an ON state. That is, at most one of the plurality of second electrodes e2 is capable of being in communication with the output terminal of the first multiplex selection module 10 and outputting a sensing electrical signal.

Among the plurality of first bias switches S21, each first bias switch S21 can be connected between a corresponding second electrode e2 and the bias voltage generation module 20. When the first bias switch S21 is turned on, it is equivalent to communicating the corresponding second electrode e2 with the bias voltage generation module 20, and in this case, the second electrode e2 can receive a bias voltage.

It should be noted that at any time, in order to avoid the second electrode e2 corresponding to the redundant nanopore np from being in the floating state, the first bias switches S21 corresponding to the remaining second electrodes e2 except for the second electrode e2 in communication with the output terminal of the first multiplex selection module 10 are all in an ON state; that is, all the remaining second electrodes e2 receive a bias voltage and are not in the floating state.

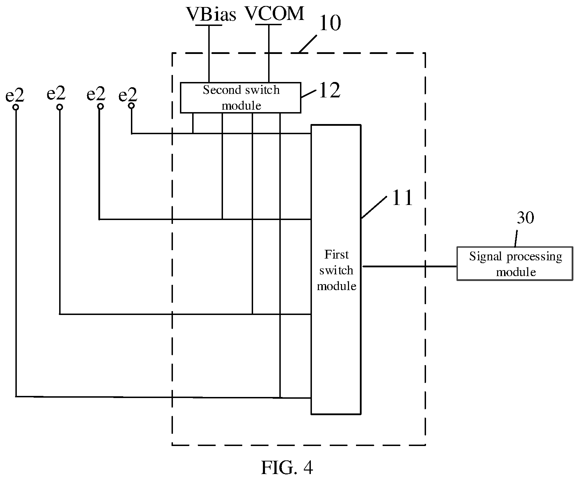

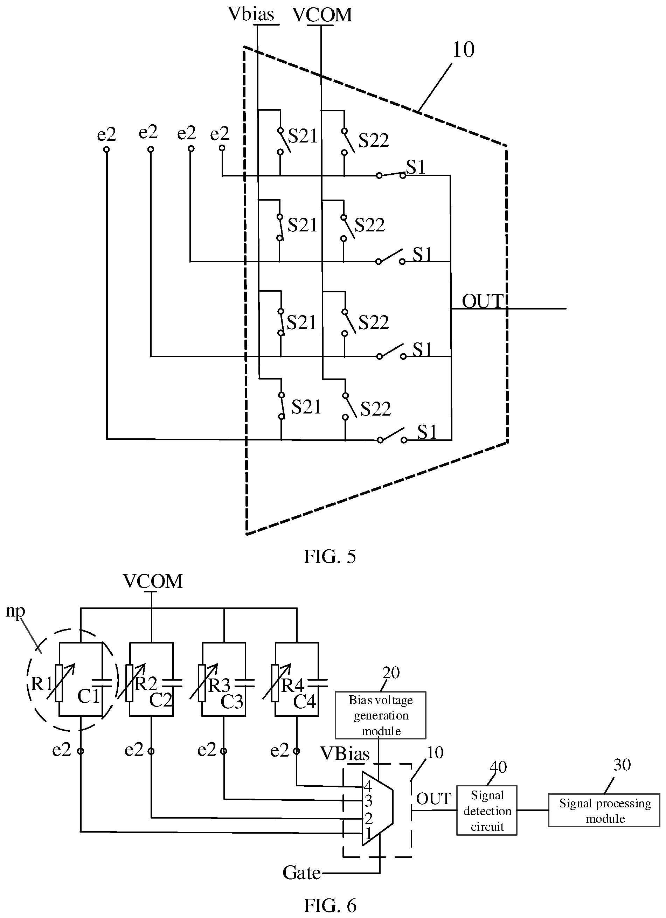

Referring to FIGs. 4 and 5, in some embodiments, the second switch module 12 described above further includes a third terminal, and the third terminal of the second switch module 12 can be connected to the common voltage terminal VCOM. The second switch module 12 further includes a plurality of second bias switches S22.

Among the plurality of second bias switches S22, each second bias switch S22 can be connected between a corresponding second electrode e2 and the common voltage terminal VCOM. When the second bias switch S22 is turned on, it is equivalent to communicating the corresponding second electrode e2 with the common voltage terminal VCOM, and in this case, the second electrode e2 can receive a common voltage.

Taking a certain second electrode e2 as an example, when the first bias switch S21 corresponding to the second electrode e2 is turned on, the second electrode e2 can receive a bias voltage, and when the second bias switch S22 corresponding to the second electrode e2 is turned on, the second electrode e2 can receive a common voltage. To distinguish the function of the first bias switch S21 from the function of the second bias switch S22, the bias voltage generated by the bias voltage generation module 20 may be set to be less than the common voltage provided by the common voltage terminal VCOM. That is, when the first bias switch S21 is turned on, the second electrode e2 receives a bias voltage less than the common voltage. In this case, there is a certain potential difference between the first electrode e1 and the second electrode e2, and the potential difference can realize the reduction process of the electrode or the reduction process of ions in the electrolyte, such that the metal electrode or electrolyte consumed during sequencing can be repaired, thereby prolonging the service life of the electrode or electrolyte. When the second bias switch S22 is turned on, the voltage received by the second electrode e2 is a common voltage, and in this case, the potential difference between the first electrode e1 and the second electrode e2 is substantially 0, such that the analyte to be detected cannot be driven to pass through the nanopore np corresponding to the second electrode e2.

Referring to FIG. 6, in some embodiments, the sensor device described above may further include a signal detection circuit 40.

The signal detection circuit 40 can be connected between the first multiplex selection module 10 and the signal processing module 30. The signal detection circuit 40 can perform signal amplification on a sensing electrical signal from the first multiplex selection module 10.

The signal detection circuit 40 can receive the sensing electrical signal output by the output terminal of the first multiplex selection module 10, and perform signal amplification and signal conversion processing on the sensing electrical signal, thereby converting the sensing electrical signal into a signal type that can be acquired by the signal processing module 30. For example, the power supply voltage of the signal detection circuit 40 may be greater than the common voltage provided by the common voltage terminal VCOM, and in this case, the signal detection circuit 40 may perform signal amplification processing on the sensing electrical signal, thereby increasing the amplitude of the sensing electrical signal with a low amplitude to be within the voltage range that can be detected by the signal processing module 30. The signal detection circuit 40 may further perform analog-to-digital conversion on the sensing electrical signal to convert an analog signal into a digital signal that can be recognized by the signal processing module 30.

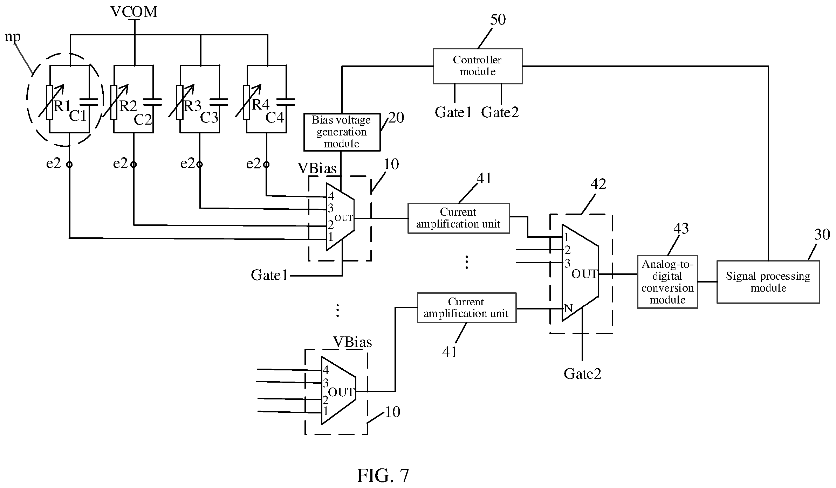

Referring to FIG. 7, in some embodiments, the sensor device described above may include a plurality of first multiplex selection modules 10, and the signal detection circuit 40 of the sensor device may include a plurality of current amplification units 41. Further, the sensor device may further include a second multiplex selection module 42 and an analog-to-digital conversion module 43.

Due to the limited number of gates that can be achieved by a single first multiplex selection module 10, it is generally necessary to provide a plurality of first multiplex selection modules 10 when there are a large number of nanopores np on the membrane. For example, the first multiplex selection module 10 may include 4 first signal input terminals, and each first multiplex selection module 10 may be connected to the second electrodes e2 corresponding to 4 nanopores np. When there are a large number of nanopores np on the membrane, for example, when 20 nanopores np are provided on the membrane, at least 5 first multiplex selection modules 10 need to be provided.

When the sensor device is provided with a plurality of first multiplex selection modules 10, the signal detection circuit 40 may include a plurality of current amplification units 41.

The plurality of current amplification units 41 may correspond to the plurality of first multiplex selection modules 10, respectively. An input terminal of each current amplification unit 41 may be connected to the corresponding first multiplex selection module 10, and the current amplification unit 41 can perform signal amplitude amplification processing on a sensing electrical signal output by the corresponding first multiplex selection module 10, such that the sensing electrical signal can be within the sampling range of the signal processing module 30 after the amplification processing. The current amplification unit 41 can also perform noise reduction and filtering on the sensing electrical signal.

It can be understood that if the sensing electrical signal output by the second electrode e2 to the output terminal of the first multiplex selection module 10 is already within the sampling range of the signal processing module 30 before being subjected to signal amplification, the current amplification unit 41 can be omitted.

The common voltage provided by the common voltage terminal VCOM can be determined according to the power supply voltage of the current amplification unit 41. That is, the common voltage should be set between the highest potential and the lowest potential that can be provided by the current amplification unit 41. For example, when the power supply voltage of the current amplification unit 41 is 3.3 V, the common voltage may be set to any voltage value between 0 V and 3.3 V, such as 0.5 V, 0.8 V, 1.0 V, 1.2 V, 1.5 V, 2.0 V, or 2.5 V. The common voltage is usually set to 1.6 V.

The second multiplex selection module 42 can be connected to output terminals of the plurality of current amplification units 41. The second multiplex selection module 42 can select one current amplification unit 41 from the plurality of current amplification units 41 and output the sensing electrical signal amplified by the current amplification unit 41.

An input terminal of the analog-to-digital conversion module 43 can be connected to an output terminal of the second multiplex selection module 42, and an output terminal of the analog-to-digital conversion module 43 is connected to the input terminal of the signal processing module 30. The analog-to-digital conversion module 43 can convert the amplified sensing electrical signal output by the second multiplex selection module 42 from an analog signal to a digital signal, such that the signal processing module 30 can analyze and process the sensing electrical signal by reading the digital signal.

As an exemplary implementation, 20 nanopores np are provided on the membrane, the sensor device includes 5 first multiplex selection modules 10, and each first multiplex selection module 10 can be connected to the second electrodes e2 of 4 nanopores np. The 5 first multiplex selection modules 10 are connected to the second multiplex selection module 42 through 5 current amplification units 41, respectively. The second multiplex selection module 42 can be selectively in communication with one of the 5 first multiplex selection modules 10, and the communicated first multiplex selection module 10 can be selectively in communication with one of the second electrodes e2 of the corresponding 4 nanopores np. The selective conduction between the first multiplex selection module 10 and the second multiplex selection module 42 can achieve the selective communication of the 20 nanopores np; that is, one of the plurality of nanopores np is selected for a nanopore np sequencing process.

With continued reference to FIG. 7, in some embodiments, the sensor device described above may further include a controller module 50.

The controller module 50 includes an input terminal and a gate signal terminal. As shown in FIG. 7, the input terminal of the controller module 50 can be connected to an output terminal of the signal processing module 30, and the gate signal terminal Gate1 of the controller module 50 can be connected to the first multiplex selection module 10.

The controller module 50 can generate a gate signal corresponding to one of the plurality of second electrodes e2 and send the gate signal to the first multiplex selection module 10. The first multiplex selection module 10 communicates the corresponding second electrode e2 with the output terminal of the first multiplex selection module 10 according to the gate signal to output a sensing electrical signal, and connects the remaining second electrodes e2 with the bias voltage generation module 20 or the common voltage terminal VCOM, such that the second electrodes e2 that do not output a sensing electrical signal receive a bias voltage or a common voltage.

In some embodiments, the controller module 50 described above can be connected to the bias voltage generation module 20.

In the case that there is at least one nanopore np among the plurality of nanopores np in a damaged state and the second switch module does not include a second bias switch S22, since the second switch module only includes the first bias switch S21, the sensor device can only provide a bias voltage for the second electrode e2, but cannot provide a common voltage. In this case, if the bias voltage is less than the common voltage, the potential difference between the common voltage and the bias voltage may generate a large current on the damaged nanopore np, causing further damage to the damaged nanopore np and the surrounding normal nanopore np. Therefore, when there is a nanopore np in a damaged state and only a bias voltage can be provided for the second electrode e2, the controller module 50 can drive the bias voltage generation module 20 to generate a bias voltage equal to the common voltage, such that the potential difference between the common voltage and the bias voltage is substantially 0, thereby avoiding the generation of a large instantaneous current on the damaged nanopore np to cause further damage.

In some other embodiments, if there is at least one nanopore np among the plurality of nanopores np in a damaged state and the second switch module includes a second bias switch S22, since the second switch module includes the first bias switch S21 and the second bias switch S22, the controller module 50 can drive the first multiplex selection module 10 to switch the second electrode e2 corresponding to the nanopore np in the damaged state to be connected to the common voltage terminal VCOM. In this case, among the plurality of nanopores np not in communication with the output terminal of the first multiplex selection module 10, a nanopore np in a normal state can receive a bias voltage by means of the first bias switch S21 that is turned on, while the nanopore np in the damaged state can receive a common voltage by means of the second bias switch S22 that is turned on. The potential difference between the first electrode e1 and the second electrode e2 at the two ends of the damaged nanopore np is substantially 0, such that no large instantaneous current is generated in the damaged nanopore np, and further damage can be avoided.

It should be noted that in the above embodiments, the controller module 50 can send a bias voltage control signal to control the bias voltage generation module 20 to generate an appropriate bias voltage.

When each of the redundant nanopores np is a nanopore np in a normal state, the controller module 50 can control the bias voltage generation module 20 to generate a bias voltage less than the common voltage, and in this case, the potential difference between the bias voltage and the common voltage can repair ions in the consumed electrode or electrolyte. When there is at least one nanopore np among the redundant nanopores np in a damaged state, since the equivalent resistance value of the damaged nanopore np is greatly reduced compared with that of the normal nanopore np, in order to avoid generating a large instantaneous current pulse on the damaged nanopore np, the controller module 50 can control the bias voltage generation module 20 to generate a bias voltage consistent with the common voltage; in this case, the potential difference between the bias voltage and the common voltage is 0 or close to 0, and the small potential difference does not generate a large current on the damaged nanopore np, such that the damaged nanopore np and the surrounding normal nanopore np are not further damaged, and the nanopore np sequencing process is not affected.

In some embodiments, an implementation of the signal processing module 30 acquiring the state of each nanopore np may be driving the first multiplex selection module 10 to sequentially connect each second electrode e2 to the output terminal of the first multiplex selection module 10 before selecting a certain nanopore np for sequencing.

When each second electrode e2 is connected to the output terminal of the first multiplex selection module 10, the signal processing module 30 can receive a sensing electrical signal corresponding to the second electrode e2. When the analyte to be detected does not pass through the nanopore np, the normal nanopore np may be equivalent to a large resistance, and in this case, the sensing electrical signal received by the signal processing module 30 should be an empty pore current.

The equivalent resistance value of the damaged nanopore np is greatly reduced compared with that of the normal nanopore np. Therefore, when the signal processing module 30 connects the second electrode e2 corresponding to the damaged nanopore np to the output terminal of the first multiplex selection module 10, the received sensing electrical signal will be greater than the empty pore current. The signal processing module 30 can separately determine that the state of each nanopore np is a normal state or a damaged state according to the magnitudes of the currents of the sensing electrical signals corresponding to different second electrodes e2.

After the states of the nanopores np are sequentially determined, if there is at least one nanopore np in a damaged state, in order to avoid a large pulse current generated when the potential difference between the common voltage and the bias voltage is applied to the nanopore np in the damaged state, the controller module 50 can drive the bias voltage generation module 20 to generate a first bias voltage, and the first bias voltage can be consistent with the common voltage. That is, when there is a nanopore np in a damaged state, the bias voltage cannot be set to be less than the common voltage, but should be set to be consistent with the common voltage to avoid generating a large current on the damaged nanopore np and affecting the accuracy of nanopore np sequencing.

In the above embodiments, the sensing switch S1, the first bias switch S21, and the second bias switch S22 may be an analog switch integrated circuit, a single metal-oxide-semiconductor field-effect transistor (MOSFET) in an integrated circuit, or a complementary switch circuit composed of two different types of transistors.

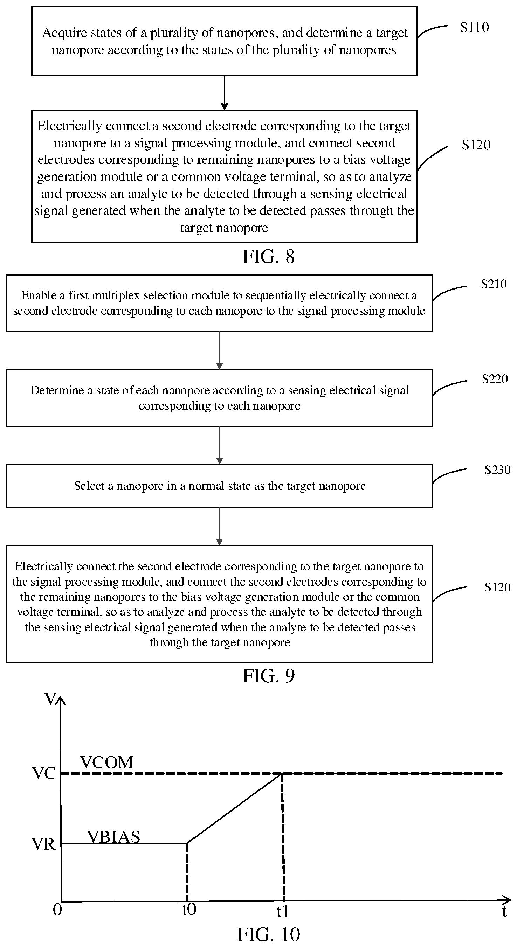

Referring to FIG. 8, the embodiments of the present application further provide a method for operating a sensor device, applied to the sensor device in the above embodiments. The method includes the following steps.

In S110, states of a plurality of nanopores are acquired, and a target nanopore is determined according to the states of the plurality of nanopores.

In S120, a second electrode corresponding to the target nanopore is electrically connected to the signal processing module, and second electrodes corresponding to remaining nanopores are connected to the bias voltage generation module or the common voltage terminal, so as to analyze and process an analyte to be detected through a sensing electrical signal generated when the analyte to be detected passes through the target nanopore.

The method for operating a sensor device provided in the embodiments of the present application is applied to the sensor device in the above embodiments. The sensor device can select one of the plurality of nanopores to analyze and process the analyte to be detected, and the other redundant nanopores not subjected to analysis and processing can be connected to the bias voltage generation module or the common voltage terminal to provide a bias voltage or a common voltage for the redundant nanopores, such that the second electrodes of the redundant nanopores are not in a floating state and can provide stable charge bleeding channels, thereby avoiding excessive charge accumulation on the membrane to affect the accuracy of nanopore sequencing or cause damage to the membrane and the nanopores.

In this embodiment, by determining the states of the plurality of nanopores, the target nanopore can be determined from the nanopores in a normal state. The second electrode of the target nanopore is electrically connected to the signal processing module, the analyte to be detected is driven to pass through the target nanopore by the potential difference between the two ends of the target nanopore, and the analyte to be detected is analyzed and processed according to the sensing electrical signal generated in the process of the analyte to be detected passing through the target nanopore. For example, the gene sequence of the analyte to be detected can be deduced according to the sensing electrical signal, thereby achieving nanopore sequencing. For the other redundant nanopores not subjected to sequencing, the second electrode corresponding to each redundant nanopore can be connected to the bias voltage generation module or the common voltage terminal to provide a bias voltage or a common voltage for the redundant nanopore, so as to prevent the second electrode of the redundant nanopore from being in a floating state for a long time, thereby forming an effective charge bleeding channel, and prevent the sequencing process from being interfered with by a large instantaneous current generated during nanopore switching.

In S110, based on the sensor device in the above embodiments, analysis and processing can be performed on the analyte to be detected in the chamber.

When the analysis and processing are performed, the controller module of the sensor device can acquire the states of the plurality of nanopores and determine the target nanopore according to the states of the plurality of nanopores.

Generally, to improve the efficiency of nanopore sequencing, a redundant nanopore array mode may be adopted; that is, a plurality of nanopores are provided on the membrane, one nanopore can be selected from the plurality of nanopores as the target nanopore, and the nanopore is connected to a sequencing circuit to achieve gene sequencing of the analyte to be detected. After determining the states of the plurality of nanopores, the controller module can select the target nanopore from the nanopores in a normal state, while a nanopore in a damaged state does not serve as the target nanopore.

Referring to FIG. 9, as an optional embodiment, the step S110 described above may include the following steps.

In S210, the first multiplex selection module is enabled to sequentially electrically connect a second electrode corresponding to each nanopore to the signal processing module.

In S220, a state of each nanopore is determined according to a sensing electrical signal corresponding to each nanopore.

In S230, a nanopore in a normal state is selected as the target nanopore.

In this embodiment, the controller module can control the first multiplex selection module to sequentially electrically connect the second electrode of each nanopore to the signal processing module, and can determine that each nanopore is in a normal state or a damaged state according to the sensing electrical signal corresponding to each nanopore. After the state of each nanopore is determined, one nanopore from a plurality of nanopores in a normal state may be selected as the target nanopore.

In S210, the controller module may detect the state of each nanopore in a manner of sequentially connecting the second electrode of each nanopore to the signal processing module by means of the first multiplex selection module to construct a sequencing circuit by means of each nanopore. In S220, since a nanopore in a normal state may be equivalent to a resistance with a large value, the resistance value of a damaged nanopore is significantly reduced. By electrically connecting the second electrode of each nanopore to the signal processing module, the sensing electrical signal of each nanopore can be acquired. The equivalent resistance range of each nanopore can be determined according to the sensing electrical signal corresponding to each nanopore. If the sensing electrical signal of a certain nanopore is too large, it indicates that the equivalent resistance of the nanopore is small, and the nanopore may be damaged.

Whether the state of each nanopore is normal can be determined by determining whether the sensing electrical signal corresponding to each nanopore is in an appropriate current range.

It should be noted that the above process of performing state detection on each nanopore is before the sequencing process of the analyte to be detected, and at this time, no analyte to be detected is added into the chamber, and the equivalent resistance of the nanopore remains stable. In S230, after the states corresponding to the plurality of nanopores are determined, the nanopores in a normal state can be determined from the plurality of nanopores, and one of the nanopores in a normal state is selected as the target nanopore.

In S120, after the target nanopore is selected from the plurality of nanopores, the second electrode corresponding to the target nanopore can be electrically connected to the signal processing module; that is, the first multiplex selection module is controlled to connect the second electrode corresponding to the target nanopore to the output terminal of the first multiplex selection module, and connect the second electrodes corresponding to the remaining nanopores to the bias voltage generation module or the common voltage terminal.

The first electrode of the target nanopore is provided with a common voltage, and a potential difference between the first electrode and the second electrode will drive the analyte to be detected to pass through the target nanopore from the first chamber to the second sub-chamber corresponding to the target nanopore. In the process of the analyte to be detected passing through the target nanopore, the blockage of the nanopore by bases of the analyte to be detected will cause a change in the equivalent resistance of the target nanopore, thereby causing a change in the sensing electrical signal received by the signal processing module. The signal processing module can deduce the types of the bases passing through the target nanopore according to the detected sensing electrical signal, thereby determining the gene sequence of the analyte to be detected and realizing the sequencing of the analyte to be detected.

As an optional embodiment, the method for operating the sensor device may further include the following steps.

In S310, in the case that the second electrode corresponding to the target nanopore is electrically connected to the signal processing module, a state of the target nanopore is monitored according to a sensing electrical signal generated by the target nanopore.

In S320, when the target nanopore is damaged, the second electrode corresponding to the target nanopore is switched to be connected to the bias voltage generation module or the common voltage terminal.

In this embodiment, in the process of analyzing and processing the analyte to be detected by means of the target nanopore, the state of the target nanopore can also be monitored according to the sensing electrical signal generated by the target nanopore. When the target nanopore is damaged, it is necessary to replace the nanopore with a new one for analysis and processing, and switch the second electrode corresponding to the target nanopore to be connected to the bias voltage generation module or the common voltage terminal. When the target nanopore is damaged,

In S310, after determining the target nanopore, the sensor device can electrically connect the second electrode corresponding to the target nanopore to the signal processing module. When the second electrode corresponding to the target nanopore is electrically connected to the signal processing module, the sensor device can further monitor the state of the target nanopore according to the sensing electrical signal generated by the target nanopore.

The signal processing module in the sensor device can not only analyze and process the analyte to be detected according to the sensing electrical signal generated by the target nanopore, but also determine whether the target nanopore is in a damaged state according to the sensing electrical signal generated by the target nanopore. In the process of analyzing and processing the analyte to be detected by means of the target nanopore, it is also necessary to monitor the state of the target nanopore and switch to a normal nanopore in a timely manner when the target nanopore is damaged.

As an optional implementation, when no analyte to be detected passes through the nanopore, the sensing electrical signal of the nanopore is usually about 100 pA, and in this case, the normal current range of the nanopore is an empty pore current range; when an analyte to be detected passes through the nanopore, the sensing electrical signal of the nanopore is reduced compared with the empty pore current and is only a fraction of the empty pore current, and in this case, the normal current range of the nanopore is a sequencing current range.

If the sensing electrical signal remains within the empty pore current range and the sequencing current range, it indicates that the target nanopore is in a normal state. If the sensing electrical signal is beyond the empty pore current range, it indicates that the equivalent resistance of the target nanopore is too small at this time, and the target nanopore can be determined to be in a damaged state.

In S320, when the state of the target nanopore is monitored according to the sensing electrical signal generated by the target nanopore, if the sensing electrical signal abnormally increases beyond a preset range, it can be determined that the target nanopore is damaged, and in this case, the sensor device can switch the second electrode corresponding to the target nanopore to be connected to the bias voltage generation module or the common voltage terminal.

When the target nanopore is damaged, the analysis and processing of the analyte to be detected can no longer be performed by means of the target nanopore, and it is necessary to switch to another normal nanopore. The sensor device can disconnect the damaged target nanopore from the output terminal of the first multiplex selection module and connect the damaged target nanopore to the bias voltage generation module or the common voltage terminal, such that the target nanopore in a damaged state becomes a redundant nanopore. By providing a bias voltage or a common voltage, the redundant nanopore can be prevented from being in a floating state. It should be noted that since the target nanopore is in a damaged state, if the second electrode corresponding to the target nanopore is connected to the common voltage terminal, it can be ensured that the potential difference between the two ends of the target nanopore is substantially 0, and a large current pulse will not be generated on the target nanopore in the damaged state; if the second electrode corresponding to the target nanopore needs to be connected to the bias voltage generation module, the bias voltage provided by the bias voltage generation module also needs to be adjusted to be consistent with the common voltage.

In some embodiments, in the process of replacing the target nanopore with a new one for analysis and processing when the target nanopore is damaged, if the bias voltage is inconsistent with the common voltage, the voltage change rate of the bias voltage should be set to be less than a preset slope value in the time interval when the bias voltage is increased to be consistent with the common voltage; that is, the voltage change rate of the bias voltage is avoided from being too high, such that the instantaneous current peak value in the process of changing the bias voltage is reduced.

As shown in FIG. 10, before time t0, the bias voltage at the bias voltage terminal is VR, and the bias voltage VR is less than the common voltage VC provided by the common voltage terminal. At this time, the electrode or the electrolyte corresponding to the redundant nanopore connected to the bias voltage terminal is in the reduction stage. The switching of the target nanopore starts at time t0. In the interval from t0 to t1, the controller module can control the bias voltage generation module to increase the bias voltage VR to the common voltage VC. In the process of increasing the bias voltage, the voltage change rate of the bias voltage is as follows: $\frac{VC - VR}{t 1 - t 0} .$

To avoid a too high voltage change rate of the bias voltage, the preset slope value SR1 may be set, and when the controller module controls the bias voltage generation module to increase the bias voltage VR, the following condition should be met: $\frac{VCOM - VR}{t 1 - t 0} \leq SR 1 .$

That is, the voltage change rate may be limited to avoid a too large instantaneous current peak value in the process of changing the bias voltage.

The embodiments of the present application further provide a nanopore sequencing apparatus. The nanopore sequencing apparatus includes the sensor device in the above embodiments and a membrane including a plurality of nanopores. An analyte to be detected is added into the first chamber divided by the membrane, and nanopore sequencing of the analyte to be detected can be achieved by the sensor device.

The functional blocks shown in the structural block diagrams described above may be implemented as hardware, software, firmware, or a combination thereof. When implemented in hardware, it may be, for example, an electronic circuit, an application-specific integrated circuit (ASIC), appropriate firmware, a plug-in, a function card, or the like. When implemented in software, the elements of the present application are programs or code segments used to perform required tasks. The programs or code segments may be stored in a machine-readable medium or transmitted over a transmission medium or communication link via a data signal carried in a carrier wave. A "machine-readable medium" may include any medium capable of storing or transmitting information. Examples of machine-readable media include electronic circuits, semiconductor memory devices, ROMs, flash memories, erasable ROMs (EROMs), floppy disks, CD-ROMs, optical disks, hard disks, fiber optic media, radio frequency (RF) links, and the like. The code segments may be downloaded via a computer network such as the Internet, an intranet, or the like.

It should be noted that the terms "comprise", "include", or any other variants thereof herein are intended to cover a non-exclusive inclusion, such that a process, method, article, or device that includes a list of elements includes not only those elements but also other elements not explicitly listed or inherent to such process, method, article, or device.

The principle and embodiments of the present application are described with specific examples herein, and the above examples are merely intended to help understand the method and core idea of the present application. The above descriptions are only optional implementations of the present application. It should be noted that due to the limited nature of written expression and the objectively unlimited specific structures, those of ordinary skill in the art can also make several improvements, modifications, or changes without departing from the principle of the present application, and can also combine the above technical features in an appropriate manner. Such improvements, modifications, changes, or combinations, or direct application of the concepts and technical solutions of the present application to other scenarios without modifications, should be considered within the protection scope of the present application.

## Claims

1. A sensor device, wherein the sensor device comprises:
a chamber, wherein in a case that the chamber is provided with a membrane comprising a plurality of nanopores, the chamber is divided into a first chamber and a second chamber by the membrane, the second chamber comprises a plurality of second sub-chambers separated from each other, and each of the second sub-chambers is in communication with the first chamber through a corresponding nanopore;
a first electrode, disposed in the first chamber, wherein the first electrode is connected to a common voltage terminal;
a plurality of second electrodes, disposed in the plurality of second sub-chambers, respectively, wherein a potential difference between the first electrode and each of the second electrodes is used to drive an analyte to be detected to pass through a corresponding nanopore and generate a sensing electrical signal;
a bias voltage generation module, configured to generate a bias voltage, wherein the bias voltage is less than or equal to a common voltage provided by the common voltage terminal;
a first multiplex selection module, connected to the plurality of second electrodes, wherein the first multiplex selection module is capable of selecting one second electrode from the plurality of second electrodes to be in communication with an output terminal of the first multiplex selection module to output a sensing electrical signal, and connecting second electrodes that are not in communication with the output terminal of the first multiplex selection module to the bias voltage generation module or the common voltage terminal; and
a signal processing module, wherein an input terminal of the signal processing module is connected to the first multiplex selection module, and the signal processing module is capable of acquiring a state of a nanopore according to a sensing electrical signal output by the first multiplex selection module.

2. The sensor device according to claim 1, wherein the first multiplex selection module comprises:
a first switch module, wherein a plurality of first terminals of the first switch module are connected to the plurality of second electrodes, respectively, and a second terminal of the first switch module is connected to the output terminal of the first multiplex selection module; the first switch module is configured to output a sensing electrical signal of one of the plurality of second electrodes; and
a second switch module, wherein a plurality of first terminals of the second switch module are connected to the plurality of second electrodes, respectively, and a second terminal of the second switch module is connected to the bias voltage generation module.

3. The sensor device according to claim 2, wherein the first switch module comprises:
a plurality of sensing switches, wherein each of the sensing switches is connected between a corresponding second electrode and the output terminal of the first multiplex selection module;
at any time, at most one of the plurality of sensing switches is in an ON state;
the second switch module comprises:
a plurality of first bias switches, wherein each of the first bias switches is connected between a corresponding second electrode and the bias voltage generation module.

4. The sensor device according to claim 3, wherein a third terminal of the second switch module is connected to the common voltage terminal; the second switch module further comprises:
a plurality of second bias switches, wherein each of the second bias switches is connected between a corresponding second electrode and the common voltage terminal, wherein the bias voltage generated by the bias voltage generation module is less than the common voltage provided by the common voltage terminal.

5. The sensor device according to claim 1, wherein the sensor device further comprises:
a signal detection circuit, wherein the signal detection circuit is connected between the first multiplex selection module and the signal processing module, and is configured to perform signal amplification on a sensing electrical signal from the first multiplex selection module.

6. The sensor device according to claim 5, wherein the sensor device comprises a plurality of first multiplex selection modules;
the signal detection circuit comprises:
a plurality of current amplification units, wherein an input terminal of each of the current amplification units is connected to a corresponding first multiplex selection module; the current amplification unit is configured to perform signal amplification on a sensing electrical signal output by the corresponding first multiplex selection module;
the sensor device further comprises:
a second multiplex selection module, wherein the second multiplex selection module is connected to output terminals of the plurality of current amplification units, and is configured to output an amplified sensing electrical signal from one of the plurality of current amplification units; and
an analog-to-digital conversion module, wherein an input terminal of the analog-to-digital conversion module is connected to an output terminal of the second multiplex selection module, and an output terminal of the analog-to-digital conversion module is connected to the input terminal of the signal processing module.

7. The sensor device according to claim 3 or 4, wherein the sensor device further comprises:
a controller module, wherein an input terminal of the controller module is connected to an output terminal of the signal processing module, and a gate signal terminal of the controller module is connected to the first multiplex selection module; the controller module is configured to generate a gate signal corresponding to one of the plurality of second electrodes and send the gate signal to the first multiplex selection module.

8. The sensor device according to claim 7, wherein the controller module is connected to the bias voltage generation module;
in a case that there is at least one nanopore in a damaged state and the second switch module does not comprise a second bias switch, the controller module is configured to drive the bias voltage generation module to generate a bias voltage equal to the common voltage.

9. The sensor device according to claim 7, wherein in a case that there is at least one nanopore in a damaged state and the second switch module comprises a second bias switch, the controller module is configured to drive the first multiplex selection module to switch a second electrode corresponding to the nanopore in the damaged state to be connected to the common voltage terminal.

10. A method for operating a sensor device, applied to the sensor device according to any one of claims 1 to 9, the method comprising:
acquiring states of the plurality of nanopores, and determining a target nanopore according to the states of the plurality of nanopores; and
electrically connecting a second electrode corresponding to the target nanopore to the signal processing module, and connecting second electrodes corresponding to remaining nanopores to the bias voltage generation module or the common voltage terminal, so as to analyze and process an analyte to be detected through a sensing electrical signal generated when the analyte to be detected passes through the target nanopore.

11. The method for operating a sensor device according to claim 10, wherein acquiring the states of the plurality of nanopores and determining the target nanopore according to the states of the plurality of nanopores, comprises:
enabling the first multiplex selection module to sequentially electrically connect a second electrode corresponding to each nanopore to the signal processing module;
determining a state of each nanopore according to a sensing electrical signal corresponding to each nanopore; and
selecting a nanopore in a normal state as the target nanopore.

12. The method for operating a sensor device according to claim 10, wherein the method for operating a sensor device further comprises:
monitoring, according to a sensing electrical signal generated by the target nanopore, a state of the target nanopore in a case that the second electrode corresponding to the target nanopore is electrically connected to the signal processing module; and
when the target nanopore is damaged, switching the second electrode corresponding to the target nanopore to be connected to the bias voltage generation module or the common voltage terminal.
